# EUROPÄISCHE PATENTANMELDUNG

(11) **EP 2 039 394 A1**
(43) Veröffentlichungstag der Anmeldung: **25.03.2009**
(21) Anmeldenummer: 08163471.9
(22) Anmeldetag: 02.09.2008
(51) Int. Cl.: A61N 5/10, G06F 19/00

(54) **Anlage sowie Verfahren zum Betreiben einer Anlage**

(30) Priorität: 19.09.2007 DE 102007044630
(71) Anmelder: Siemens Aktiengesellschaft, 80333 München (DE)
(72) Erfinder: Schneider, Michael, 91054 Erlangen (DE)

(57) **Zusammenfassung**

Die Erfindung betrifft eine Anlage, insbesondere Partikeltherapieanlage, umfassend:
- mehrere räumlich verteilte, konfigurierbare Systeme, die jeweils derart ausgebildet sind, dass eine Einstellung bei einem der konfigurierbaren Systeme mithilfe von Konfigurationsdaten umsetzbar ist,
- einen zentralen Konfigurationsserver, auf dem die Konfigurationsdaten zentral gespeichert sind, und
- eine Vernetzung der konfigurierbaren Systeme mit dem zentralen Konfigurationsserver

wobei
- jedes der konfigurierbaren Systeme eine lokale Kopie der Konfigurationsdaten, die auf dem zentralen Konfigurationsserver gespeichert sind, aufweist, und
- die konfigurierbaren Systeme derart ausgebildet sind, dass zu der Umsetzung einer Einstellung auf lokal gespeicherte Konfigurationsdaten zugegriffen wird, die in der lokalen Kopie der Konfigurationsdaten hinterlegt sind.

Weiterhin betrifft die Erfindung ein Verfahren zum Betreiben einer derartigen Anlage.

## Beschreibung

Die Erfindung betrifft eine Anlage, insbesondere eine Partikeltherapieanlage, in der mehrere räumlich verteilte konfigurierbare Systeme zum Einsatz kommen. Weiterhin betrifft die Erfindung ein Verfahren zum Betreiben einer derartigen Anlage.

In einer Anlage, in der mehrere räumlich verteilte und konfigurierbare Systeme zum Einsatz kommen, fallen üblicherweise zahlreiche Konfigurationsdaten für diese konfigurierbaren Systeme an, die sich teilweise überschneiden. Eine derartige Anlage kann beispielsweise eine Partikeltherapieanlage sein.

Eine Partikeltherapieanlage wird zur Behandlung insbesondere von Tumorerkrankungen eingesetzt. Üblicherweise ist eine Partikeltherapieanlage eine Anlage mit zahlreichen Räumen. Partikel, die zur Bestrahlung eingesetzt werden, werden dabei üblicherweise in einer Partikelquelle erzeugt, von einem Partikelbeschleuniger auf die zur Bestrahlung notwendigen Energien beschleunigt und über ein Strahltransportsystem zu einem gewünschten Behandlungsraum geführt, wo letztlich die Bestrahlung und die Behandlung eines Patienten durchgeführt wird.

In einer derartigen Anlage kommen üblicherweise verschiedenste Systeme zum Einsatz, durch deren Zusammenspiel der korrekte Betrieb der Anlage gewährleistet wird. Bei vielen dieser konfigurierbaren Systeme müssen dabei bestimmte vordefinierte Einstellungen umgesetzt werden, um sie in einem Betriebsmodus zu versetzen, der das Betreiben der Anlage ermöglicht. Hierzu müssen die Systeme konfiguriert werden können, d.h. den konfigurierbaren Systemen muss ein Zugriff auf bestimmte Konfigurationsdaten ermöglicht werden. Üblicherweise werden hierfür IT-Systeme, d.h. rechnergestützte Systeme, eingesetzt.

Eine einfache Möglichkeit, eine derartige Anlage zu betreiben, ist es, Konfigurationsdaten jedem einzelnen konfigurierbaren System bereitzustellen, beispielsweise dadurch, dass ein Anwender die Konfigurationsdaten an jedem einzelnen System eingibt.

Eine weitere Möglichkeit, eine Partikeltherapieanlage zu betreiben, ist in der US 2004/0164254 A1 offenbart. In dem dort offenbarten Konfigurations-Management-System ist eine Datenbank beschrieben, die Daten und Konfigurationsparameter umfasst, und eine Management-Komponente, mit deren Hilfe spezielle Systemsteuerungsdateien aus den Untermengen der Daten der Datenbank erzeugt werden und zu ausgewählten Geräten, beispielsweise Magnetsysteme, gesendet werden, so dass eine Konfiguration dieser ausgewählten Geräte ermöglicht wird. Mithilfe der Systemsteuerungsdateien sollen sogenannte Single-Point-of-Failure-Ereignisse in der Datenbank abgefangen werden und ein sicherer Betrieb gewährleistet werden.

Die Tatsache, dass mehrere konfigurierbare Systeme und Komponenten in einer Anlage zum Einsatz kommen, ist jedoch nicht nur auf Partikeltherapieanlagen beschränkt, sondern findet sich ebenfalls bei anderen Anlagen, insbesondere Industrieanlagen, wie beispielsweise Kraftwerke, Fertigungsanlagen, usw. In all diesen Anlagen kommen räumlich verteilte und konfigurierbare Systeme zum Einsatz, bei denen abhängig von einem gewünschten Betriebsmodus bestimmte Einstellungen an den konfigurierbaren Systemen umgesetzt werden müssen.

Es ist daher die Aufgabe der Erfindung, eine Anlage mit mehreren räumlich verteilten und konfigurierbaren Systemen bereitzustellen, bei der eine Wartung und Konfiguration der konfigurierbare Systeme auf einfache Weise erfolgen kann und mit der gleichzeitig ein sicherer und stabiler Betrieb möglich ist. Weiterhin ist die Aufgabe der Erfindung, ein Verfahren zum Betreiben einer derartigen Anlage bereitzustellen, durch das eine Wartung und Konfiguration der konfigurierbaren Systeme auf einfache und sichere Weise erfolgen kann bei gleichzeitig stabilen Betrieb der Anlage.

Demnach wird die Aufgabe der Erfindung durch eine Anlage gemäß Anspruch 1 gelöst sowie durch ein Verfahren zum Betreiben einer Anlage gemäß Anspruch 6. Vorteilhafte Weiterbildungen der Erfindung finden sich in den Merkmalen der abhängigen Ansprüche.

Die erfindungsgemäße Anlage, insbesondere eine Partikeltherapieanlage, umfasst:
- mehrere räumlich verteilte, konfigurierbare Systeme, die jeweils derart ausgebildet sind, dass eine Einstellung bei einem der konfigurierbaren Systeme mithilfe von Konfigurationsdaten umsetzbar ist,
- einen zentralen Konfigurationsserver, auf dem die Konfigurationsdaten zentral gespeichert sind, und
- eine Vernetzung der konfigurierbaren Systeme mit dem zentralen Konfigurationsserver,
   wobei
- jedes der konfigurierbaren Systeme eine lokale Kopie der Konfigurationsdaten, die auf dem zentralen Konfigurationsserver gespeichert sind, aufweist, und
- die konfigurierbaren Systeme derart ausgebildet sind, dass zu der Umsetzung der Einstellung auf lokal gespeicherte Konfigurationsdaten zugegriffen wird, die in der lokalen Kopie der Konfigurationsdaten hinterlegt sind.

Dies bedeutet, dass ein konfigurierbares System auf diejenigen Konfigurationsdaten zur Umsetzung einer Einstellung zugreift, die in der lokalen Kopie der Konfigurationsdaten dieses konfigurierbaren Systems hinterlegt sind.

Eine derart ausgebildete Anlage weist den Vorteil auf, dass Konfigurationsdaten redundant vorhanden sind. Sollte ein Ausfall des zentralen Konfigurationsservers eintreten, kann die Anlage dennoch in weiten Teilen weiterhin betrieben werden, da die konfigurierbaren Systeme zur Umsetzung einer Einstellung durch Konfigurationsdaten auf die jeweils lokal vorhandene Kopie der Konfigurationsdaten zugreift. Ausfälle der Anlage, die auf einem Ausfall bzw. auf einem Fehler des zentralen Konfigurationsservers beruhen (so genannte "single point of failure"-Ereignisse), werden hierdurch vermieden.

Ein konfigurierbares System umfasst dabei üblicherweise ein IT-System, mit dessen Hilfe Konfigurationsdaten rechnergestützt umgesetzt werden können.

Da die Konfigurationsdaten bei den konfigurierbaren Systemen als lokale Kopie von zentral gespeicherten Konfigurationsdaten vorhanden sind, ist ebenso gewährleistet, dass die konfigurierbaren Systeme auf einen identischen Satz von Konfigurationsdaten zugreifen. Insbesondere wenn die konfigurierbaren Systeme untereinander vernetzt sind, ist auf diese Weise eine in sich konsistente Kommunikation der konfigurierbaren Systeme untereinander möglich. Fehler, die sich daraus ergeben, dass konfigurierbare Systeme auf unterschiedliche Konfigurationsdaten zugreifen, werden hierdurch vermieden.

Insbesondere sind bei der Anlage der zentrale Konfigurationsserver und die konfigurierbaren Systeme derart ausgebildet, dass bei einer Änderung der Konfigurationsdaten eine Synchronisation der geänderten Konfigurationsdaten mit den Konfigurationsdaten, die in den lokalen Kopien gespeichert sind, dadurch durchführbar ist, indem
- in einem ersten Schritt die geänderten Konfigurationsdaten von dem zentralen Konfigurationsserver jeweils zu den konfigurierbaren Systemen gesendet werden,
- in einem zweiten Schritt der Erhalt der geänderten Konfigurationsdaten von den jeweiligen konfigurierbaren Systemen bestätigt wird, und
- in einem dritten Schritt ein Update der lokalen Kopien mit den geänderten Konfigurationsdaten erst dann erfolgt, nachdem alle konfigurierbaren Systeme den Erhalt der geänderten Konfigurationsdaten bestätigt haben.

Auf diese Weise wird ein einfaches und sicheres Ändern und Synchronisieren der Konfigurationsdaten, beispielsweise durch einen Anwender, auf dem zentralen Konfigurationsserver ermöglicht. Falls ein Anwender die Konfigurationsdaten einer lokalen Kopie der Kompensationsdaten ändert, kann die Änderung anschließend dem zentralen Konfigurationsserver weitergeleitet werden und daraufhin das Update der Konfigurationsdaten in den lokalen Kopien wie beschrieben durchgeführt werden.

Auf diese Weise ist gewährleistet, dass ein Anwender Konfigurationsdaten an einer zentralen Stelle ändern kann, und dass ein Update-Vorgang mit diesen geänderten Konfigurationsdaten sicher durchgeführt wird, derart, dass die konfigurierbaren Systeme stets auf einen identischen Satz von Konfigurationsdaten zugreifen. So können identische Einstellungen leicht identifiziert und konsolidiert werden, d.h., dass man nur an einer Stelle bzw. an einem IT-System einen Konfigurationseintrag vornehmen muss. Der Fall, dass nur ein Teil der konfigurierbaren Systeme geänderte Konfigurationsdaten erhalten, und dass versehentlich an verschiedenen Systemen unterschiedliche Konfigurationsdaten vorliegen, wird hierdurch vermieden. Konfigurationsdaten werden zwischen verschiedenen konfigurierbaren Systemen auf einfache Weise konsolidiert.

Wenn eines der konfigurierbaren Systeme ausfällt und ersetzt werden muss, kann das konfigurierbare System auf einfache Weise mit allen notwendigen Konfigurationsdaten versehen werden, indem eine lokale Kopie der Konfigurationsdaten des zentralen Konfigurationsservers bei dem konfigurierbaren System erstellt wird. Die Anlage ist auch in analoger Weise um weitere konfigurierbare Systeme erweiterbar. Wird zu einem späteren Zeitpunkt ein neues konfigurierbares System zugekauft und der Anlage hinzugefügt, können die auf dem zentralen Konfigurationsserver gespeicherten Konfigurationsdaten dem neuen konfigurierbaren System zur Verfügung gestellt werden.

Dadurch, dass die Konfigurationsdaten an einer zentralen Stelle geändert werden, können leicht Abhängigkeiten zwischen den Konfigurationsdaten berücksichtigt werden. Wenn die Auswahl bzw. Abänderung von bestimmten Konfigurationsdaten zur Folge hat, dass sich der Wertebereich für andere Konfigurationsdaten anderer Systeme ändert, kann eine Überprüfung dieser Abhängigkeit an einer einzigen zentralen Stelle leicht realisiert werden. Eine versehentliche Eingabe fehlerhafter Konfigurationsdaten kann so wirksam abgefangen werden.

Anstelle eines zentralen Konfigurationsservers kann auch eines der konfigurierbaren Systeme als eigenständiges konfigurierbares System mit einer lokalen Installation des Servers eingesetzt werden. In diesem Falle übernimmt das eigenständig konfigurierbare System die Funktion des zentralen Konfigurationsservers. Diese Ausgestaltung eignet sich insbesondere dann, wenn die Anlage erweiterbar ausgelegt ist und zunächst mit nur einem konfigurierbaren System betrieben werden soll. Dieses konfigurierbare System kann später als zentraler Konfigurationsserver für weitere hinzukommende konfigurierbare Systeme dienen.

Insbesondere können die Konfigurationsdaten sowohl bei dem Konfigurationsserver als auch bei den konfigurierbaren Systemen in einer Datenbank gespeichert sein. In diesem Fall weist jedes der konfigurierbaren Systeme eine lokale Kopie der zentral gespeicherten Datenbank auf.

Zusätzlich zu den konfigurierbaren Systemen kann die Anlage weitere konfigurierbare Systeme aufweisen, die zur Umsetzung einer Einstellung direkt auf die zentral gespeicherten Konfigurationsdaten des zentralen Konfigurationsservers zurückgreifen.

Insbesondere bei einer als Partikeltherapieanlage ausgebildeten Anlage kommen derartige konfigurierbare Systeme vorteilhaft zum Einsatz. Eine derartige Partikeltherapieanlage umfasst eine Partikelquelle zur Erzeugung von Partikeln, eine Beschleunigereinheit zur Beschleunigung der Partikel und zur Formung eines Partikelstrahls, zumindest einen Bestrahlungsplatz, sowie eine Partikelstrahlzuführungseinheit zum Führen des Partikelstrahls von der Beschleunigereinheit zu dem zumindest einen Bestrahlungsplatz. Die Beschleunigereinheit und/oder die Partikelstrahlzuführungseinheit werden dabei von einer Steuerungseinheit gesteuert. Die Steuerungseinheit - oder eine Komponente der Steuerungseinheit - ist dabei als ein konfigurierbares System ausgebildet, das eine lokale Kopie der zentral gespeicherten Konfigurationsdaten aufweist. Zur Steuerung der Beschleunigereinheit und/oder der Partikelstrahlzuführungseinheit greift die Steuerungseinheit bzw. die Komponente der Steuerungseinheit auf die lokal gespeicherten Konfigurationsdaten zu. Die Steuerungseinheit gewährleistet anhand einer Übermittlung von ausgewählten Konfigurationsdaten an die Beschleunigereinheit bzw. Partikelstrahlzuführungseinheit eine korrekte Strahlführung zu einem der vorhandenen Bestrahlungsplätze und/oder eine gewünschte Strahlqualität.

Bei dem erfindungsgemäßen Verfahren zum Betreiben einer Anlage, insbesondere einer Partikeltherapieanlage, mit mehreren räumlich verteilten und konfigurierbaren Systemen, die mit einem zentralen Konfigurationsserver vernetzt sind, wobei eine Einstellung bei einem der konfigurierbaren Systeme mithilfe von Konfigurationsdaten umgesetzt wird,
- wird bei jedem der konfigurierbaren Systeme eine lokale Kopie der Konfigurationsdaten, die auf dem zentralen Konfigurationsserver gespeichert sind, erzeugt, und
- die Einstellung bei einem der konfigurierbaren Systeme dadurch umgesetzt, indem auf die Konfigurationsdaten zugegriffen wird, die in der lokalen Kopie der Konfigurationsdaten dieses konfigurierbaren Systems hinterlegt sind.

Bei einer Veränderung der auf dem zentralen Konfigurationsserver gespeicherten Konfigurationsdaten wird in einer vorteilhaften Ausführungsform eine Synchronisation der geänderten Konfigurationsdaten mit den lokalen Kopien der Konfigurationsdaten durchgeführt, indem
- die geänderten Konfigurationsdaten von dem zentralen Konfigurationsserver jeweils zu den konfigurierbaren Systemen gesendet werden,
- der Erhalt der geänderten Konfigurationsdaten jeweils von den konfigurierbaren Systemen bestätigt wird,
- ein Update der lokalen Kopien mit den geänderten Konfigurationsdaten erst dann erfolgt, nachdem alle konfigurierbaren Systeme den Erhalt der geänderten Konfigurationsdaten bestätigt haben.

Insbesondere können beim Betreiben der Anlage die auf dem zentralen Konfigurationsserver gespeicherten Konfigurationsdaten durch einen Anwender geändert werden.

Ausführungsformen der Erfindung mit vorteilhaften Weiterbildungen gemäß den Merkmalen der unabhängigen Ansprüche werden nun anhand der folgenden Zeichnung näher erläutert, ohne jedoch darauf beschränkt zu sein. Es zeigen:
- Fig. 1: einen schematischen Überblick über eine Partikelthera- pieanlage, in der verschiedene konfigurierbare Syste- me zum Einsatz kommen,
- Fig. 2: einen schematischen Überblick über ein Verfahren zum Betreiben einer Anlage mit mehreren konfigurierbaren Systemen.

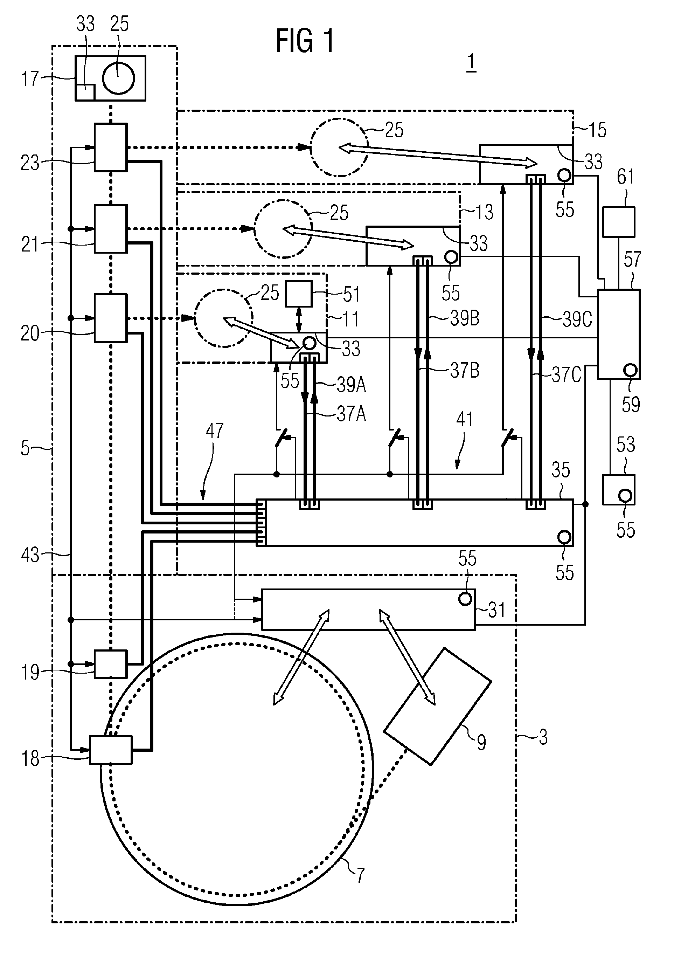

Fig. 1 zeigt schematisch den Aufbau einer als Partikeltherapieanlage 1 aufgebauten Anlage. In der Partikeltherapieanlage 1 kommen verschiedene unterschiedliche Systeme zum Einsatz, deren Zusammenspiel das korrekte Betreiben der Partikeltherapieanlage 1 ermöglicht.

Die Partikeltherapieanlage 1 weist eine Beschleunigereinheit 3 und eine Partikelstrahlzuführungseinheit 5 auf. Beispielhaft für einen Beschleuniger wurde ein Synchrotron 7 mit einer vorgeschalteten Linearbeschleunigereinheit 9 dargestellt. Die Partikelstrahlzuführungseinheit 5 führt die beschleunigten und zu einem Strahl geformten Partikel zu einem von mehreren Bestrahlungsplätzen 11 ... 15 oder zu einem Überprüfungsplatz 17.

Beispielhaft sind schematisch drei Behandlungsplätze 11, 13 und 15 zur Strahlentherapie und ein Überprüfungsplatz 17 zur Gewährleistung der Qualität des Partikelstrahls angedeutet. Am Überprüfungsplatz 17 erfolgt beispielsweise die Qualitätssicherung mit Hilfe von Qualitätsprozeduren.

Mithilfe einer Auskoppelvorrichtung 18 werden in der Beschleunigereinheit 3 - z.B. in einem Synchrotron - beschleunigte Partikel ausgekoppelt und in die Partikelstrahlzuführungseinheit 5 eingekoppelt. Die Möglichkeit einer schnellen Strahlabschaltung nach Beendigung oder Unterbrechung des Bestrahlungsvorgangs erfolgt beispielsweise durch den Einbau einer Schikane 19, die nach der Extraktionseinheit 18 angeordnet ist.

Die Zuführung der Partikel zu den Bestrahlungsplätzen 11, 13 und 15 erfolgt durch die Ablenkung des Partikelstrahls mittels Umlenkmagnete 20, 21 und 23 aus einer Hauptstrahlrichtung in der Partikelstrahlzuführungseinheit 5. In Hauptstrahlrichtung befindet sich der Überprüfungsplatz 17. An den Bestrahlungsplätzen 11, 13, 15 und am Überprüfungsplatz 17 erfolgt die Wechselwirkung der Partikel mit einem zu bestrahlenden Patienten oder einem Phantom in Bestrahlungszonen 25. Eine der Bestrahlungszonen 25 ist z.B. durch einen maximal abscanbaren Scanbereich einer (Raster-)Scanvorrichtung, einen maximal bestrahlbaren Scatterbereich einer Scattervorrichtung oder einen einstellbaren Gantry-Bestrahlungsbereich etc. gegeben.

In der Partikeltherapieanlage 1 kommen mehrere konfigurierbare Systeme zum Einsatz.

Eines dieser konfigurierbaren Systeme ist beispielsweise eine Komponente einer Steuerungseinheit für die Beschleunigereinheit 3 und/oder die Partikelstrahlzuführungseinheit 5 der Partikeltherapieanlage 1. Die Steuerungseinheit ist in der hier gezeigten Partikeltherapieanlage 1 auf zwei Komponenten aufgeteilt. Eine derartige Komponente ist beispielsweise eine Beschleunigerkontrolleinheit 31. Eine weitere derartige Komponente ist beispielsweise eine Zuordnungseinheit 35. Die Funktionsweise dieser Komponenten wird später näher erläutert.

Die Steuerungseinheit 31, 35 für die Beschleunigereinheit 3 und/oder die Partikelstrahlzuführungseinheit 5 sorgt u.a. dafür, dass nur derjenige Bestrahlungsplatz 11 ... 15 bzw. Überprüfungsplatz 17 einen Partikelstrahl zugeführt bekommt, der ihn auch angefordert hat und dass ein angeforderter Partikelstrahl einer vorgegebenen Spezifikation an dem Bestrahlungsplatz 11 ... 15 bzw. Überprüfungsplatz 17 ankommt.

Weitere konfigurierbare Systeme der Partikeltherapieanlage sind Kontrolleinheiten 33, von denen sich jeweils eine an einem Bestrahlungsplatz 11 ... 15 bzw. Überprüfungsplatz 17 befindet. Eine derartige Kontrolleinheit 33 sorgt dafür, dass der Ablauf eines Bestrahlungsvorgangs entsprechend einer Bestrahlungsplanung an dem zugeordneten Bestrahlungsplatz durchgeführt wird.

Weitere konfigurierbare Systeme sind Bestrahlungsplanungsplätze 53, an denen ein Anwender eine vorzunehmende Bestrahlung planen kann und einen Bestrahlungsplan 51 erstellen kann. Ein derartiger Bestrahlungsplan wird den Kontrolleinheiten 33 übermittelt.

Um ein korrektes Funktionieren der Partikeltherapieanlage 1 zu gewährleisten, sind die konfigurierbaren Systeme miteinander vernetzt und kommunizieren miteinander. Der Übersichtlichkeit halber ist eine Vernetzung nicht durchgehend eingezeichnet. Ein jedes dieser konfigurierbaren Systeme ist befähigt, bestimmte Komponenten der Partikeltherapieanlage 1 entsprechend bestimmter Konfigurationsdaten zu konfigurieren.

Beispielsweise sorgen die Beschleunigerkontrolleinheit 31 und die Zuordnungseinheit 35 dafür, dass ein angeforderter Partikelstrahl im richtigen Bestrahlungsplatz 11 ... 15 entsprechend seiner Spezifikation ankommt. Hierfür werden Konfigurationsdaten von der Beschleunigerkontrolleinheit 31 und von der Zuordnungseinheit 35 an die konfigurierbaren Strahlführungselemente gesendet. Eine Umsetzung einer gewünschten Konfiguration der Strahlführungselemente bewirkt, dass eine korrekte Strahlführung mit den gewünschten Spezifikationen gewährleistet ist. Zu den Strahlführungselementen gehören beispielsweise die Komponenten der Partikelstrahlzuführungseinheit 5 sowie der Beschleunigereinheit 3, wie z.B. die Extraktionseinheit 18, die Schikane 19, sowie die Umlenkmagnete 20, 21, 23.

Die Zuordnungseinheit 35 sendet hierzu ein Aktivierungssignal an die zur korrekten Strahlführung notwendigen Strahlführungselemente über fest zugeordnete Signalverbindungen 47, wie z.B. zu den entsprechenden Umlenkmagneten 20, 21, 23. Sobald das Aktivierungssignal an den Strahlführungselementen anliegt, können Konfigurationsdaten, die von der Beschleunigerkontrolleinheit 31 an die Strahlführungselemente über ein Datenbussystem 43 gesendet werden, umgesetzt werden. Hierdurch werden die zu Strahlführung notwendigen Strahlführungselemente entsprechend eingestellt.

Die Zuordnungseinheit 35 ihrerseits erhält von einer Kontrolleinheit 33 eines Bestrahlungsplatzes 11 ... 15 ein Anforderungssignal über eine feste und eindeutig zugeordnete Signalleitung 37A ... 37C, 39A ... 39C. Hierdurch wird der Zuordnungseinheit 35 in eindeutiger Weise mitgeteilt, welcher Behandlungsraum 33 den Partikelstrahl anfordert.

Um zu gewährleisten, dass die konfigurierbaren Systeme kompatibel miteinander kommunizieren können, müssen Konfigurationsdaten, auf die die konfigurierbaren Systeme zugreifen, untereinander konsistent sein.

Um dies zu erreichen, weist jedes dieser konfigurierbaren Systeme eine lokale Datenbank 55 auf, in der Konfigurationsdaten hinterlegt sind. Die lokalen Datenbanken 55 sind lokale Kopien einer zentralen, auf einem Konfigurationsserver 57 gespeicherten Datenbank 59. Der zentrale Konfigurationsserver weist ein User-Interface 61 auf, über das ein Anwender Einstellungen an dem zentralen Konfigurationsserver 57 vornehmen kann.

Die Konfigurationsdaten, die die Zuordnungseinheit 35 als Aktivierungssignal an die zur korrekten Strahlführung notwendigen Strahlführungselemente sendet, oder die Konfigurationsdaten, die die Beschleunigerkontrolleinheit 31 an die Strahlführungselemente zur korrekten Einstellung der Strahlführungselemente sendet, sind beispielsweise in den jeweils lokalen Datenbanken 55 hinterlegt, die der Zuordnungseinheit 35 bzw. der Beschleunigerkontrolleinheit 31 zugeordnet sind.

Dadurch, dass auch die anderen konfigurierbaren Systeme jeweils eine lokale Datenbank 55 aufweisen, die eine Kopie der zentralen Datenbank 59 ist, greifen alle konfigurierbaren Systeme auf einen identischen Satz von Konfigurationsdaten zu. Hierdurch ist gewährleistet, dass Konfigurationsdaten untereinander konsistent sind, wenn die konfigurierbaren Systeme miteinander direkt kommunizieren. Eine derartige Kommunikation findet beispielsweise statt, wenn ein Bestrahlungsplatz 11 ... 15 einen Partikelstrahl anfordert, oder wenn Spezifikationen für einen Partikelstrahl aus einem Therapieplan 51 durch eine Kontrolleinheit 33 über ein weiteres Datenbussystem 41 an die Beschleunigerkontrolleinheit 31 übermittelt werden.

Dadurch, dass die konfigurierbaren Systeme auf die lokalen Datenbanken 55 zugreifen, ist ein sicheres Betreiben der Partikeltherapieanlage 1 auch dann möglich, wenn die zentrale Datenbank 59 oder eine Verbindung mit der zentralen Datenbank 59 zeitweise nicht zur Verfügung steht. Das sichere Betreiben einer Partikeltherapieanlage 1 wird dadurch erhöht.

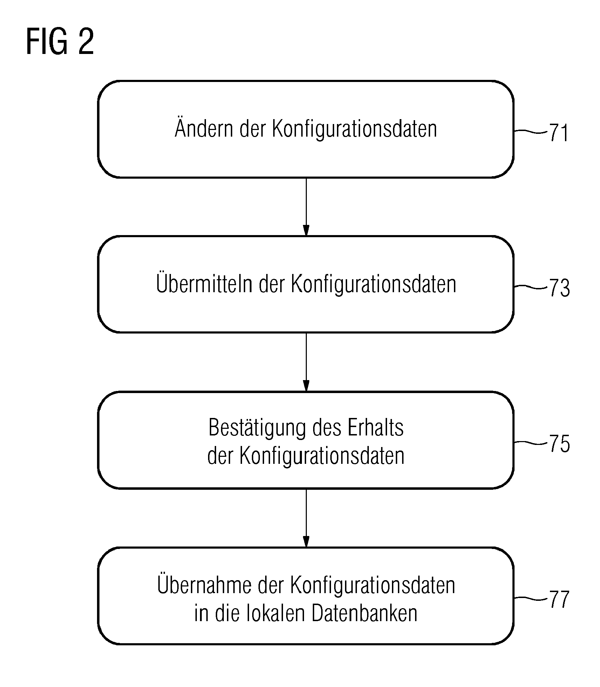

Fig. 2 zeigt Verfahrensschritte, die durchgeführt werden, wenn eine Änderung von Konfigurationsdaten vorgenommen wird. Die Änderung der Konfigurationsdaten (Schritt 71) kann dabei an der zentralen Datenbank 59 direkt vorgenommen werden. Für den Fall, dass die Änderung der Konfigurationsdaten an einer der lokalen Datenbanken 55 vorgenommen wird, werden die geänderten Konfigurationsdaten der zentralen Datenbank 59 übermittelt.

Anschließend werden die geänderten Konfigurationsdaten von dem zentralen Konfigurationsserver 57 den konfigurierbaren Systemen, d.h. der Zuordnungseinheit 35, der Beschleunigerkontrolleinheit 31, den Kontrolleinheiten 33, dem Bestrahlungsplanungsplatz 53, etc. übermittelt (Schritt 73). Nach der Übermittlung der geänderten Konfigurationsdaten wird der Erhalt der geänderten Konfigurationsdaten von konfigurierbaren Systemen bestätigt (Schritt 75). Erst nach erfolgreicher Bestätigung des korrekten Erhalts der geänderten Konfigurationsdaten durch sämtliche konfigurierbaren Systeme werden die übermittelten geänderten Konfigurationsdaten umgesetzt und in die lokalen Datenbanken 55 aufgenommen (Schritt 77). Hierdurch ist gewährleistet, dass alle Datenbanken, d.h. sowohl die zentrale Datenbank 59 als auch die lokalen Datenbanken 57 stets über denselben Satz von Konfigurationsdaten verfügen. Hierdurch werden wirksam unterschiedliche Datenbankversionen vermieden, selbst wenn Konfigurationsdaten durch einen Anwender geändert werden.

## Patentansprüche

1. Anlage, insbesondere Partikeltherapieanlage (1), umfassend:
- mehrere räumlich verteilte, konfigurierbare Systeme (31, 33, 35, 53), die jeweils derart ausgebildet sind, dass eine Einstellung bei einem der konfigurierbaren Systeme mithilfe von Konfigurationsdaten umsetzbar ist,
- einen zentralen Konfigurationsserver (57), auf dem die Konfigurationsdaten zentral gespeichert sind, und
- eine Vernetzung der konfigurierbaren Systeme (31, 33, 35, 53), mit dem zentralen Konfigurationsserver (57)
wobei
- jedes der konfigurierbaren Systeme (31, 33, 35, 53) eine lokale Kopie der Konfigurationsdaten, die auf dem zentralen Konfigurationsserver (57) gespeichert sind, aufweist, und
- die konfigurierbaren Systeme (31, 33, 35, 53) derart ausgebildet sind, dass zu der Umsetzung einer Einstellung bei einem der konfigurierbaren Systeme auf Konfigurationsdaten zugegriffen wird, die in der zugeordneten lokalen Kopie der Konfigurationsdaten hinterlegt sind.

2. Anlage nach Anspruch 1, wobei
der zentrale Konfigurationsserver (57) und die konfigurierbaren Systeme (31, 33, 35, 53) derart ausgebildet sind, dass bei einer Änderung der Konfigurationsdaten eine Synchronisation der geänderten Konfigurationsdaten mit den lokalen Kopien der Konfigurationsdaten durchführbar ist, indem
- in einem ersten Schritt die geänderten Konfigurationsdaten von dem zentralen Konfigurationsserver (57) jeweils zu den konfigurierbaren Systemen (31, 33, 35, 53) gesendet werden,
- in einem zweiten Schritt der Erhalt der geänderten Konfigurationsdaten von den jeweiligen konfigurierbaren Systemen (31, 33, 35, 53) bestätigt wird, und
- in einem dritten Schritt ein Update der lokalen Kopien mit den geänderten Konfigurationsdaten erst dann erfolgt, nachdem alle konfigurierbaren Systeme (31, 33, 35, 53) den Erhalt der geänderten Konfigurationsdaten bestätigt haben.

3. Anlage nach Anspruch 1 oder 2, wobei
die Konfigurationsdaten auf dem zentralen Konfigurationsserver (57) in einer Datenbank (59) gespeichert sind, und wobei jedes der konfigurierbaren Systeme eine lokale Kopie (55) der Datenbank aufweist.

4. Anlage nach einem der Ansprüche 1 bis 3, wobei die Anlage zumindest ein zusätzliches konfigurierbares System aufweist, das mit dem zentralen Konfigurationsserver (57) vernetzt ist, und wobei das zusätzliche konfigurierbare System derart ausgebildet sind, dass eine Einstellung bei dem zusätzlichen konfigurierbaren System umsetzbar ist, indem auf die zentral gespeicherten Konfigurationsdaten des zentralen Konfigurationsservers (57) zugegriffen wird.

5. Anlage nach einem der Ansprüche 1 bis 4, wobei die Anlage als Partikeltherapieanlage (1) ausgebildet ist, die Partikeltherapieanlage (1) umfassend
- eine Partikelquelle zur Erzeugung von Partikeln,
- eine Beschleunigereinheit (3) zur Beschleunigung der Partikel und zur Formung eines Partikelstrahls,
- zumindest einen Bestrahlungsplatz (11 ... 17),
- eine Partikelstrahlzuführungseinheit (5) zum Führen des Partikelstrahls von der Beschleunigereinheit (3) zu dem zumindest einen Bestrahlungsplatz (11 ... 17),
wobei
eines der konfigurierbaren Systeme der Partikeltherapieanlage eine Steuerungseinheit (31, 35) für die Beschleunigereinheit (3) und/oder die Partikelstrahlzuführungseinheit (5) ist, welche Steuerungseinheit (31, 35) anhand einer Übermittlung von lokal gespeicherten Konfigurationsdaten an die Beschleunigereinheit (3) bzw. Partikelstrahlzuführungseinheit (5) eine korrekte Strahlführung zu dem zumindest einen Bestrahlungsplatz (11 ... 17) und/oder eine gewünschte Strahlqualität gewährleistet.

6. Verfahren zum Betreiben einer Anlage, insbesondere Partikeltherapieanlage (1), mit mehreren räumlich verteilten und konfigurierbaren Systemen (31, 33, 35, 53), die mit einem zentralen Konfigurationsserver (57) vernetzt sind,
indem
- bei jedem der konfigurierbaren Systeme (31, 33, 35, 53) eine lokale Kopie der Konfigurationsdaten, die auf dem zentralen Konfigurationsserver gespeichert sind, erzeugt wird, und
- eine Einstellung bei einem der konfigurierbaren Systeme (31, 33, 35, 53) umgesetzt wird, indem auf die Konfigurationsdaten zugegriffen wird, die in der lokalen Kopie der Konfigurationsdaten des konfigurierbaren Systems (31, 33, 35, 53) hinterlegt sind.

7. Verfahren nach Anspruch 6, wobei
bei einer Veränderung der auf dem zentralen Konfigurationsserver (57) gespeicherten Konfigurationsdaten eine Synchronisation der geänderten Konfigurationsdaten mit den lokalen Kopien der Konfigurationsdaten durchgeführt wird, indem
- die geänderten Konfigurationsdaten von dem zentralen Konfigurationsserver (57) jeweils zu den konfigurierbaren Systemen (31, 33, 35, 53) gesendet werden,
- der Erhalt der geänderten Konfigurationsdaten jeweils von den konfigurierbaren Systemen (31, 33, 35, 53) bestätigt wird, und
- ein Update der lokalen Kopien mit den geänderten Konfigurationsdaten erst dann erfolgt, nachdem alle konfigurierbaren Systeme (31, 33, 35, 53) den Erhalt der geänderten Konfigurationsdaten bestätigt haben.

8. Verfahren nach Anspruch 6 oder 7, bei dem
die Anlage als Partikeltherapieanlage (1) ausgebildet ist, die Partikeltherapieanlage (1) umfassend
- eine Partikelquelle zur Erzeugung von Partikeln,
- eine Beschleunigereinheit (3) zur Beschleunigung der Partikel und zur Formung eines Partikelstrahls,
- zumindest einen Bestrahlungsplatz (11 ... 17),
- eine Partikelstrahlzuführungseinheit (5) zum Führen des Partikelstrahls von der Beschleunigereinheit (3) zu dem zumindest einen Bestrahlungsplatz (11 ... 17),
und wobei
eines der konfigurierbaren Systeme (31, 33, 35, 53) zumindest eine Steuerungseinheit (31, 35) für die Beschleunigereinheit (3) und/oder die Partikelstrahlzuführungseinheit (5) ist, welche Steuerungseinheit (31, 35) eine korrekte Strahlführung des Partikelstrahls zu dem zumindest einen Bestrahlungsplatz und eine gewünschte Strahlqualität gewährleistet, indem die Steuerungseinheit (31, 35) auf Konfigurationsdaten, die in der lokalen Kopie der Konfigurationsdaten der Steuerungseinheit (31, 35) hinterlegt sind, zugreift und diese Konfigurationsdaten der Beschleunigereinheit (3) bzw. der Partikelstrahlzuführungseinheit (5) übermittelt.
